# EUROPEAN PATENT SPECIFICATION

(11) **EP 3 351 242 B1**
(45) Date of publication and mention of the grant of the patent: **01.07.2026**
(21) Application number: 16845409.8
(22) Date of filing: 16.09.2016
(51) Int. Cl.: A61K 31/352, A61K 47/44, A61K 47/10, A61K 9/08, A61P 25/00, A61P 25/08, A61P 25/16, A61P 25/18, A61P 25/20, A61P 25/04, A61P 25/22, A61P 43/00

(54) **PROCESS FOR PREPARING A CANNABINOID-CONTAINING ORAL PHARMACEUTICAL COMPOSITION**
VERFAHREN ZUR HERSTELLUNG EINER CANNABINOIDHALTIGEN ORALEN PHARMAZEUTISCHEN ZUSAMMENSETZUNG
PROCÉDÉ DE PRÉPARATION D'UNE COMPOSITION PHARMACEUTIQUE ORALE COMPRENANT UN CANNABINOÏDE

(30) Priority: 18.09.2015 BR 102015024165
(43) Date of publication of application: 25.07.2018
(73) Proprietor: Prati, Donaduzzi & Cia LTDA, 85903-630 Toledo (BR); Universidade De São Paulo, 05508-220 São Paulo - SP (BR)
(72) Inventor: DONADUZZI, Luiz, 85903-290 Toledo (BR); DONADUZZI, Carmen Maria, 85903-290 Toledo (BR); DE MENEZES, Christian Gregory Burgos, 85902-140 Toledo (BR); JUNIOR, Liberato Brum, 85901-300 Toledo (BR); FILHO, Volnei José Tondo, 85903-630 Toledo (BR); ROSA, Patricia Moura, 85905-600 Toledo (BR); CRIPPA, José Alexandre, 14025-600 Ribeirão Preto (BR); HALLAK, Jaime Eduardo Cecilio, 14025-040 Ribeirão Preto (BR); ZUARDI, Antonio Waldo, 140140-250 Ribeirão Preto (BR); GUIMARÃES, Francisco Silveira, 14040-220 Ribeirão Preto (BR)
(74) Representative: Pace Napoleone, Maria
(86) International application number: PCT/BR2016/050231
(87) International publication number: WO 2017/045053

(56) References cited:
- WO-A1-2014/200350
- WO-A1-2017/042567
- WO-A2-2012/071389
- WO-A2-2015/184127
- GB-A- 2 450 753
- US-A1- 2003 100 602
- US-A1- 2004 138 293
- US-A1- 2014 357 708
- US-B1- 6 383 513
- US-B2- 8 222 292
- ANONYMOUS: "International Association for Cannabis as Medicine", September 2005 (2005-09-01), IACM 2005 Conference, pages 1 - 72, XP002790299, Retrieved from the Internet <URL:http://www.cannabis-med.org/meeting/Leiden2005/reader.pdf> [retrieved on 20190403]
- THOMPSON GR . ET AL.: "Comparison of acute oral toxicity of cannabinoids in Rats, Dogs and Monkeys.", TOXICOLOGY AND APPLIED PHARMACOLOGY, vol. 25, no. 3, 1973, pages 363 - 372, XP024886649
- SULLIVAN J ET AL.: "Efficacy and safety of epidiolex (cannabidiol) in children and young adults with treatment-resistant epilepsy: Initial data from an expanded access program.", EPILEPSY CURRENTS, vol. 15, no. Supp. 1, January 2015 (2015-01-01), pages 532, XP055368487

## Description

### Field of the Invention

The present invention describes an oral pharmaceutical composition comprising cannabinoid(s) in an oily liquid carrier, as well as a process for its preparation.

The present invention is useful in the treatment of neurological disorders, particularly refractory epilepsy.

The present invention describes analytical method and specifications for quality control of Active Pharmaceutical Ingredient (API) and oral pharmaceutical formulation containing cannabidiol.

The present invention describes a study for safety and efficacy evaluation of oral formulation containing cannabidiol.

### Background of the Invention

The plants of the genus *Cannabis* (*Cannabis sativa, Cannabis ruderalis* and *Cannabis indica*) have various substances of interest in science and medicine, so**-called cannabinoids, for example: Δ9**-tetrahydrocannabinol (D9-**THC), Δ8**- tetrahydrocannabinol (D8-THC), tetrahydrocannabinol acid (THC-A), tetrahydrocannabivarin (THCV), tetrahydrocannabivarin acid (THCV-A), cannabidiol (CBD), cannabidiol acid (CBD-A), cannabichromene (CBC), cannabidivarin (CBDV), cannabidivarin acid (CBDV-A), cannabigerol (CBG), cannabigerol acid (CBG-A), cannabigerovarine (CBGV), cannabinol (CBN), cannabinovarin (CBNV), among others. Scientific research was able to identify a total of 85 cannabinoids isolated from *Cannabis* plants, and their use has been intensely investigated for the treatment of epilepsy, nausea, vomiting, loss of appetite, pain and inflammatory diseases, among other pathologies.

Epilepsy is the most common neurological disorder of all, being characterized by recurrent seizures, the result of excessive electrical discharges from neurons. It affects around 50 million people worldwide and approximately 40% of all patients have seizures that are not fully controlled with drugs.

The effects from this situation affect not only the health system, but also the patients and their families, since this is a disease in which individuals lose their independence, compromising their socialization and their psychological development. In addition, cognitive performance may be affected by epilepsy, as well as by current therapy.

In this context, the use of other cannabinoids has been shown to be a promising alternative for patients not only for refractory epilepsy, but also for other neurological disorders.

The scientific literature contains numerous studies on the efficacy of cannabinoids in the treatment of epilepsy. Various patent applications, for example BR 11 2012 000076 4, WO 2012/093255, BR 11 2012024480 9, WO 2009/087351 and WO 2007/148094 express such use and claim methods of treatment and medicaments.

However, such documents express such drugs very generically without detailing the aspects of the technique involved and difficulties a skilled person would encounter in developing such formulation. In addition, most of the experimental data are carried out in mice and mice, from intra-peritoneal injections, an impracticable approach in humans.

The present invention goes beyond these general teachings, describing and proving the feasibility of an oral liquid formulation constituted by cannabinoid(s).

WO 2009/020666 discloses an oral liquid composition of cannabinoids, wherein the solvent is aqueous, and has a more efficient *in vivo* absorption profile than commercial gelatin capsules.

US 7025992 describes a cannabinoid formulation as powder, which when hydrated, has its absorption facilitated by means of an emulsifier.

WO 2015/068052 describes liposomes comprising cannabinoids and terpenes.

US2014/357708 A1 describes cannabinoid liquid oral compositions comprising a surfactant which promotes self-emulsification. An abstract titled Treatment With Cbd In Oil Solution Of Drug-Resistant Pediatric Epilepsies (September 2005) describes the use of a 2.5% CBD corn oily solution in the treatment of children with traditional antiepileptic drug-resistant seizures, reporting that the low CBD doses administered resulted in improvement in consciousness and spasticity in all children patients. GB2450753 A describes an oral liquid composition comprising CBD, tetrahydrocannabidivarin, propylene glycol, peppermint oil and ethanol, for use in the treatment of schizophrenia, epilepsy or cognitive disorders such as Alzheimer's. WO2012/071389 describes stable cannabinoid compositions comprising a high purity cannabinoid (preferably (-)-**Δ9**-trans-THC), an acid (such as citric acid), an oil (preferably sesame oil), and a pharmaceutically acceptable solvent. WO2014/200350 describes CB**N**-rich plant extracts or synthetic CBN formulations used for treating neurobehavioral disorders. US6383513 describes a composition for the nasal delivery of a cannabinoid which comprises a cannabinoid in a biphasic delivery system or a cannabinoid in a microsphere delivery. Thomson Gr et al. "Comparison of acute oral toxicity of cannabinoids in Rats, Dogs and Monkeys" - Toxicology And Applied Pharmacology 1973 is a publication reporting the results of a study focused on the neurotoxicity of Δ9-THC, Δ8-THC and Cannabis extract upon oral administration in animals. US8222292 relates to an aqueous-based oral cannabinoid solution comprising water, alcohol and propylene glycol that is stable at room or refrigerated temperatures. US2003/100602 describes the therapeutic use of delta-9-tetrahydrocannabinol (THC) for patients with symptomatic HIV infection. US2004/138293 describes a composition comprising THC and CBD for use in palliative cancer therapy and as an agent having a muscle-relaxing and/or analgetic effect in neurological diseases. Sullivan et al "Efficacy and Safety of Epidiolex (Cannabidiol) in Children and Young Adults with Treatment-Resistant Epilepsy" Epilepsy Currents, vol 15, January 2015, page 532 reports results obtained in patients with a purified 98% oil-based CBD extract, of known and constant composition (Epidiolex: GW Pharmaceuticals), at a dose of 5 mq/kq/day in addition to baseline AED regimen."

The present invention differs from the others in that it is an oily media soluble formulation which promotes the bioavailability of the active ingredient when compared to the powder pharmaceutical form.

Moreover, it is important to emphasize that all the documents of the technique point to the gelatinous capsules orally as an inefficient form for administration of the cannabinoids, with aqueous solvents being used as the alternative. The present invention challenges this dogma, and shows that it is possible to provide an oily formulation with an increased bioavailability. Thus, the teachings of the present invention should be considered novel and inventive.

### Summary of the Invention

The invention is set out in the appended set of claims.

The present disclosure concerns an oral liquid composition for improving the bioavailability, stability and organoleptic properties of cannabinoid.

It is an object of the present invention to provide

a process of preparing an oral liquid composition comprising the steps of:
a. Dissolution of cannabinoid:
   i. in the oily solvent; or
   ii. in the co-solvent, followed by addition and homogenization to the oily solvent;
b. addition of the excipients and homogenization.

It is herein described the use the oral liquid composition in the treatment of neurological disorders such as refractory epilepsy, epilepsy, Parkinson's disease, schizophrenia, sleep disorders, post-traumatic disorder, anxiety, chronic pain relief, and autism.

### Detailed Description of the Invention

The examples shown herein are only intended to exemplify some of the numerous embodiments of the invention, and therefore, should not be considered to limit the teachings herein attached.

### Liquid Oral Composition Comprising Cannabinoids

For the purposes of the present invention, the term "cannabinoids" means substances capable of activating the cannabinoid receptors present in the cells. They can be chosen from endocannabinoids, phytocannabinoids and synthetic cannabinoids.

Endocannabinoids are man-made substances capable of active cannabinoid receptors. Non-limiting examples of endocannabinoids include anandamide (AEA), 2-arachidonoylglycerol (2-AG), 2-arachidonyl glyceryl ether, N-arachidonoyl dopamine (NADA), virodhamine (OAE) and a combination thereof.

Phytocannabinoids are naturally occurring cannabinoids that can be found in plants of the *Cannabis* genus. Phytocannabinoids may be present in the form of extract, isolated or synthetically reproduced compounds. Non-limiting examples of phytocannabinoids include Δ9-tetrahydrocannabinol (D9-THC), Δ8-tetrahydrocannabinol (D8-THC), tetrahydrocannabinol acid (THC-A), tetrahydrocannabivarin (THCV), tetrahydrocannabivarin acid (THCV-A) cannabidiol (CBD), cannabidiol acid (CBD-A), cannabichromene (CBC), cannabidivarin (CBDV), cannabidivarin acid (CBDV-A), cannabigerol (CBG), cannabigerol acid (CBG-A), cannabigerovarine (CBGV), cannabinol (CBN), cannabinovarin (CBNV), and combinations thereof.

Synthetic cannabinoids are compounds capable of interacting with cannabinoid receptors and are not found either endogenously or in plants. Non-limiting examples of synthetic cannabinoids include dronabinol, nabilone, rimonabant, and combinations thereof.

Within the scope of the present invention the pharmaceutically acceptable salts and acids of the above-mentioned compounds are also contemplated.

According to the present invention, the cannabinoid is cannabidiol, its pharmaceutically acceptable acid, or combinations thereof.

The concentration of the cannabinoid in the composition ranges from 5 to 500 mg/ml, more preferably from 20 to 250 mg/ml.

The oily solvent of the present invention is corn oil.

The composition optionally comprises a co-solvent to aid in the solubilization of the cannabinoid in the oily solvent.

Non-limiting examples of co-solvents include propylene glycol, glycerol, polyethylene glycol, ethanol, and combinations thereof.

Excipients used in the composition of the present invention are the excipients commonly found in the state of art and known to those skilled in the art. Non-limiting examples include antioxidants, sweeteners, flavorings, preservatives, and combinations thereof.

The antioxidant may be selected from acetylcysteine tocopherols, α-tocopherol, d-α-tocopherol, DL-α-tocopherol, ascorbic palmitate, butylhydroxyanisole (BHA), butylhydroxytoluene (BHT), lecithin, cysteine, cysteine hydrochloride, propyl gallate, ascorbic acid, iso-ascorbic acid, thioglycerol, citric acid, tartaric acid, EDTA and its salts, hydroxyquinoline sulfate, phosphoric acid, sodium metabisulphite and sodium citrate, t-butylhydroquinone (TBHQ), and combinations thereof.

The sweetening agents may be selected from sucralose, saccharin, sodium saccharin, sodium cyclamate, sorbitol, xylitol, sucrose, glycerol, neohesperidine, aspartame, and combinations thereof. The amount of sweetening agent used may be in the range of about 0.05 to about 3% by weight of the composition.

The flavoring agents may be selected from peppermint, mint, lemon, strawberry, apple , pear, peach, raspberry, plum, pineapple aroma and the like. The amount of flavoring used ranges from about 0.05 to about 5% by weight of the composition.

Preservatives may be selected from sodium benzoate, potassium sorbate, benzyl alcohol, parabens esters (p-hydroxybenzoic acids) such as methylparaben, ethylparaben, propylparaben, butylparaben, and the like, and combinations thereof. The amount of preservatives used ranges from about 0.01 to about 2% by weight.

The oral compositions described in the present invention have cannabinoid concentrations within the specifications set forth in the analytical methodology object of this patent, remaining stable for long periods of time, including the storage period and validity, without appreciable occurrence of degradations such as Δ9-tetrahydrocannabinol and cannabinol, the main psychoactive compounds.

The present invention is further an acceptable alternative for patients, for example, children and the elderly, who cannot or do have difficulties in taking tablets or because of their clinical condition at the start of the treatment, make it impossible to swallow solid pharmaceutical formulas.

### Preparing Process

The preparation process of the composition of the present invention comprises the steps of:
a. Dissolution of cannabinoid:
   i. in the oily solvent; or
   ii. in the co-solvent, followed by addition and homogenization to the oily solvent;
b. addition of the excipients and homogenization.

Dissolution of the cannabinoid in the oily solvent occurs at temperatures between 30°C and 80°C and with stirring. When a co-solvent is used, the cannabinoid is first dissolved in the co-solvent, under the same conditions described above, and then such solution is added to the oily solvent.

The excipients are added after cooling of the solution, and kept under stirring until complete solubilization.

The entire process of manufacturing and packaging the product may be carried out under an inert gas environment such as nitrogen, argon, helium or the like.

The productive process was validated considering critical points and control steps according to the table below:

| STEP | CRITICAL POINT | CONTROL |
|---|---|---|
| PREPARATION | TIME, STIRRING SPEED, SOLUBILITY AND TEMPERATURE. | - EVALUATE AT LEAST 3 PRODUCTIVE BATCHES |
| | | - FOLLOW AND REGISTER PARAMETERS IN THE ORDER OF MANUFACTURE |
| | | - AT THE END OF THE PRODUCTION PROCESS, SAMPLES ARE REMOVED FOR THE FOLLOWING ANALYSIS: DOSING, RELATED SUBSTANCES AND MICROBIOLOGICAL ANALYSIS |
| PROCESS OF TRANSFER OF THE CASTER TO THE STOCKING TANK | NOT APPLICABLE | - EVALUATE AT LEAST 3 BATCHES |
| | | - FOLLOW AND REGISTER PARAMETERS IN THE ORDER OF MANUFACTURE |
| PRIMARY | PACKAGING | - EVALUATE THE MINIMUM 3 |
| PACKAGING | SPEED | PILOT BATCHES |
| | | - FOLLOW AND REGISTER PARAMETERS IN THE ORDER OF MANUFACTURE |
| | | - DURING PRODUCTION PROCESS THE SAMPLES ARE REMOVED FOR PACKAGING VOLUME, BOTTLE OPENING AND BOTTLE LEAKAGE ANALYSIS. BESIDES, DURING VALIDATION, THE FOLLOWING ANALYZES ARE PERFORMED: DOSING, DESCRIPTION, RELATED SUBSTANCES AND MICROBIOLOGICAL ANALYSIS |

### Use of the Composition

The present invention provides a preparation process of a composition useful in the treatment of neurological disorders, such as refractory epilepsy, epilepsy, Parkinson's disease, schizophrenia, sleep disorders, post-traumatic disorder, anxiety, chronic pain relief and autism.

The effective dose of cannabinoid ranges from 1 to 50 mg/kg/day of body weight, preferably from 2.5 to 25 mg/kg/day, and may be in single dose or divided throughout the day.

### Safety and efficacy assessment for refractory epilepsy indication

After a 4-week baseline period to complete a seizures diary, research participants who meet the inclusion criteria will be randomized to enter the 17-week treatment period. The treatment period will consist of two phases: Titration and Maintenance or treatment per se. In the Titration Phase, the physicians responsible for the follow-up of the research participants will be instructed to blind prescribe the developed formulation object of this patent or placebo with adjuvant therapy to the medications that the patients have been using, starting with a volume in ml of the research solution attributed to the research participant, which would correspond to a dose of 5mg/kg/day. The solution researched should be increased in the same volume (corresponding to a dose of 5mg/kg/day of CBD) every 7 days, if there were seizures during the previous period up to the dosage of 25 mg/kg/day or occurrence of clinically significant adverse event. In the Maintenance Phase, it is intended to maintain the highest dose obtained in the stable titration period until the end of the study.

The need for dose reduction will be allowed and evaluated if the research participant shows an adverse event related to the dose of the experimental drug. Dose escalation is allowed up to the maximum dose of 25 mg/kg/day, if after determination of the "ideal" maintenance dose the patient's seizures have returned.

Inclusion criteria for patients: An approved informed consent form (ICF) by the Ethics Committee (EC) must have been signed and dated by the legal representative; Research subjects of both genders within the age range of 6 months to 18 years; Diagnosis of refractory epilepsy characterized according to International League Against Epilepsy (ILAE) classification; Research subjects who show at least 4 epileptic seizures during the 4-week Baseline Period, with an interval between seizures that does not exceed 21 days; In treatment with up to 03 concomitant AEDs at stable doses at least 1 month before the initial visit and with the expectation of remaining stable during the treatment period. The vagus nerve stimulator (VNS) is considered as an AED; Legal representative considered capable of complying with the protocol (for example, able to understand and fill in the diaries), visiting schedule or medication intake, according to the investigator's **opinion; Presence** of **encephalic** neuroimaging (Magnetic Resonance Image (MRI) or Computed Tomography (CT)) performed within the last 5 years; Without significant co-morbidities, the **physician's** judgment, according to the other criteria adopted in this Protocol and evaluations to which he;/she was subjected: clinical history, pressure, pulse and temperature measurement, physical examination, ECG, EEG and laboratory tests and Research subjects with potential to be pregnant can be included in the study provided they are in sexual abstinence or using contraceptive method considered effective.

Exclusion Criteria: Occurrence of only simple partial seizures (preserved consciousness) without motor symptomatology; History or presence of pseudo-seizures; History of attempted suicide; History of major depression; Pregnant research participant; Research subject in use of drug abuse; Hypertensive research subject; Research subject with severe dysphagia who is not using a gastric or naso-gastric tube; Research subject in drug use research that may significantly influence the metabolism of CBD, except for AED if stable for at least 1 month prior to the initial visit; Presence of any clinical or neuroimaging signal suggesting brain disorder, brain tumor, metabolic or neurodegenerative disease with quick progression; Presence of known acute and clinically significant illness at the examining physician's criteria, such as renal, hepatic, urinary, intestinal, respiratory infections; Presence of known and clinically significant chronic disease at the examining physician's criteria that may compromise study participation or pose a safety risk to the research subject; History of liver, renal, pulmonary, gastrointestinal, hematological, heart or psychiatric disease that, in the clinical criteria, could compromise the health of the research subject and/or his/her participation in the study; Hypotension or hypertension of any etiology requiring pharmacological treatment; Surgical history that may compromise the health of the research subject and/or his/her participation in the study; Research subject in regular or intermittent use of marijuana in the last 60 days prior to the initial visit; Research subject in regular or intermittent CBD-based treatment in the last 60 days prior to the initial visit; History of allergy or idiosyncratic reactions to *Cannabis Sativa* derivatives or components of the pharmaceutical formulation; ECG abnormalities considered clinically significant according to the investigator; Subjects who have participated in another clinical study less than 3 months before the date of the Initial Visit; Donation or loss of 450 ml or more of blood within 90 days (three months) prior to the date of the Initial Visit; Liver function impairment: TGO, TGP, alkaline **phosphatase and γGT values more than 3 times above the upper limit of the reference range. A result of γGT that exceeds 3 times the upper limit only may** be acceptable if it is attributable to hepatic enzyme induction caused by concomitant treatment with AED and the other liver enzymes are below 3 times the upper limit of the reference range and Research subject with clinically significant deviations from the reference range values for the following laboratory parameters: creatinine **clearance <50 ml/min, platelets <100,000/µL, or neutrophils <1800/µL.**

Validated analytical methodology and acceptable limits to ensure identity, quality and purity of the API.

| EVALUATED PARAMETER | SPECIFICATIONS | |
|---|---|---|
| DESCRIPTION | CRYSTALLINE OR NEAR WHITE POWDER | |
| SOLUBILITY | INSOLUBLE IN PURIFIED WATER, SOLUBLE IN ETHANOL 96%, CHLOROFORM AND SLIGHTLY SOLUABLE IN HEXANE | |
| IDENTIFICATION A | SAMPLE SPECTRUM SAMPLE SHOWS MAXIMUM ABSORPTION ONLY IN THE SAME WAVELENGTHS AND WITH THE SAME RELATIVE INTENSITIES OF THOSE OBSERVED IN THE STANDARD SPECTRUM | |
| IDENTIFICATION B | THE MAIN PEAK RETENTION TIME OBTAINED ON THE SAMPLE SOLUTION CHROMATOGRAM CORRESPONDS TO THE PEAK RETENTION TIME OF THE STANDARD SOLUTION (1). | |
| FUSION POINT | 63 TO 69°C | |
| SPECIFIC ROTATION | SPECIFIC OPTICAL ROTATION MUST 120 ° TO -130 ° IN RELATION TO THE ANHYDROUS SUBSTANCE | |
| WATER | < 0.5% | |
| WATER ACTIVITY | < 0.600% | |
| HEAVY METALS | ANY COLOR DEVELOPED BY THE TEST SOLUTION IS NOT MORE INTENSE THAT IT IS DEVELOPED BY THE CONTROL SOLUTION, CORRESPONDING TO NO MORE THAN 10 PPM | |
| SULPHATED ASH (2) | < 0.1% | |
| RELATED SUBSTANCES AND DEGRADATION PRODUCTS (1) | CANNABINOL | < 0.14% |
| | DELTA 9 TETRAHYDROCANNABINOL | ≤ 0.14% |
| | INESPECIFIC INDIVIDUAL IMPURITIES | ≤ 0.14% |
| | TOTAL IMPURITIES | < 0.42% |
| DOSING (1) | 98 - 102% (1) | |
| RESIDUAL SOLVENTS (1) | ACETONE | ≤ 5000 µg.g⁻¹ |
| | HEPTANE | < 5000 µg.g⁻¹ |
| | BENZENE | < 2 µg.g⁻¹ |

Validation of analytical methodology for API content and dosing.

| EVALUATED PARAMETER | SPECIFICATIONS |
|---|---|
| SPECIFICITY AND SELECTIVITY | - PROOF THAT THE PEAK QUANTIFIED IN THE CHROMATOGRAM OF THE SOLUTION TEST IS REALLY THE ANALYTE OF INTEREST (CANABIDIOL) THROUGH THE COMPARISON OF THE RETENTION TIME OF THIS PEAK WITH THE RETENTION TIME OF CANABIDIOL IN THE STANDARD SOLUTION. |
| | - PROOF THAT NO SUBSTANCE CO-ELUATES (RESOLUTION EQUAL TO OR MORE THAN 1.5) WITH THE CANABIDIOL PEAK IN THE TEST SOLUTION THROUGH THE RAW MATERIAL SAMPLES ANALYSIS SUBMITTED TO FORCED DEGRADATION AND POSTERIOR VERIFICATION OF CANABIDIOL PEAK PURITY IN THESE SOLUTIONS. THE RESULTS OF THE PEAK PURITY ARE EXPRESSED BY COMPARING THE PURITY ANGLE WITH THE PURITY THRESHOLD WHEN PURITY ANGLE VALUE IS LESS THAN THRESHOLD PURITY VALUE, PEAK IS PURE AND, ON CONTRARY, PEAK IS IMPURE. |
| LINEARITY | - CORRELATION COEFFICIENT (R) MUST BE EQUAL OR GREATER THAN 0.99. |
| | - DO NOT PRESENT TREND IN THE DISTRIBUTION OF RESIDUES. |
| | - RELATIVE STANDARD DEVIATION (RSD) BETWEEN THE 3 SAMPLES OF EACH CONCENTRATION LEVEL, AND IT SHOULD NOT EXCEED 2.0% FOR CONCENTRATION 100 µg/mL TO <1000 µg/mL. |
| INTERVAL | - THE INTERVAL OF THE ANALYTICAL METHODOLOGY WILL BE ESTABLISHED BY THE CONFIRMATION THAT THE METHOD PROVIDES PRECISION, ACCURACY AND LINEARITY SUITABLE WITHIN 80% TO 120% RANGE OF THE NOMINAL CONCENTRATION OF THE SAMPLE IN THE DOSING ANALYTICAL METHODOLOGY (200.0 µg/mL). |
| ACCURACY | - THE RECOVERY AVERAGE IN THREE LEVELS (80%, 100% AND 120% OF THE NOMINAL CONCENTRATION OF CANABIDIOL IN THE SAMPLE) MUST BE BETWEEN 98% AND 102% |
| REPEATABILITY 1^{ST} AND 2^{ND} DAY ACCURACY | RELATIVE STANDARD DEVIATION (RSD) BETWEEN SIX DETERMINATIONS SHOULD BE MINOR OR EQUAL 2.0%. |
| INTERMEDIARY ACCURACY | - RELATIVE MEAN DIFFERENCE BETWEEN FIRST REPETIBILITY MEANS AND SECOND MUST BE LESS OR EQUAL 2.0% |
| ROBUSTNESS | - METHOD WILL BE CONSIDERED ROBUST FOR SUCH VARIATIONS WHEN THE RESOLUTION BETWEEN THE CANABIDIOL PEAK AND ANY OTHER PEAK IS NOT LESS THAN 1.5 AND THE TEST SOLUTION CONTENT NOT VARY MORE THAN 2.0% OF THE CONTENT OBTAINED WITHOUT CHANGE IN ANALYTICAL METHODOLOGY. |

Validation of analytical methodology for related substances and degradation product of API

| EVALUATED PARAMETER | SPECIFICATIONS |
|---|---|
| | - PROOF THAT THE PEAK QUANTIFIED IN THE CHROMATOGRAM OF THE TEST SOLUTION IS REALLY THE ANALYTE OF INTEREST (CANABIDIOL) THROUGH THE COMPARISON OF THE RETENTION TIME OF THIS PEAK WITH THE RETENTION TIME OF CANABIDIOL IN THE STANDARD SOLUTION. |
| SPECIFICITY | - PROOF THAT ANY DEGRADATION PRODUCT CO-ELUATES (RESOLUTION EQUAL TO OR MORE THAN 1.5) WITH THE CANABIDIOL PEAK, Δ9-THC AND CBN IN THE TEST SOLUTION THROUGH THE SAMPLES ANALYSIS SUBMITTED TO FORCED DEGRADATION IN ACID, BASE, OXIDATIVE, THERMAL, PHOTOLYTIC MEDIUM, HUMIDITY AND METAL ION AND POSTERIOR VERIFICATION OF CANABIDIOL PEAK PURITY IN THESE SOLUTIONS AND POSTERIOR VERIFICATION OF CANABIDIOL PEAK PURITY, Δ9-THC AND CBN (IF FORMED) IN THESE SOLUTIONS. |
| | THE RESULTS OF THE PEAK PURITY ARE EXPRESSED BY COMPARING THE PURITY ANGLE WITH THE PURITY THRESHOLD WHEN PURITY ANGLE VALUE IS LESS THAN THRESHOLD PURITY VALUE, PEAK IS PURE AND, ON CONTRARY, PEAK IS IMPURE. |
| LINEARITY | - CORRELATION COEFFICIENT (R) MUST BE EQUAL OR GREATER THAN 0.99. |
| | - DO NOT PRESENT TREND IN THE DISTRIBUTION OF RESIDUES. |
| | - RELATIVE STANDARD DEVIATION (RSD) BETWEEN THE 3 SAMPLES OF EACH CONCENTRATION LEVEL, AND IT SHOULD NOT EXCEED 15% FOR CONCENTRATION <1µG/ML AND IT SHOULD NOT EXCEED 11.0% FOR CONCENTRATION >1 µg/mL TO <10 µg/mL AND SHOULD NOT EXCEED 7.3% FOR CONCENTRATION >10 µg/mL TO < 100 µg/mL. |
| RESPONSE FACTOR | - RELATIVE RESPONSE FACTOR WILL BE OBTAINED THROUGH THE RATIO BETWEEN THE ANGULAR COEFFICIENTS FROM IMPURITIES AND ACTIVE, OBTAINED FROM THE LINEARITY PARAMETER. |
| LIMIT OF QUANTIFICATION | - THIS PARAMETER WAS DETERMINED FROM THE RESULTS OF THE LINEARITY PARAMETER. |
| INTERVAL | - THIS PARAMETER IS ESTABLISHED BY THE CONFIRMATION THAT THE METHOD PROVIDES PRECISION, ACCURACY AND LINEARITY SUITABLE WHEN APPLIED TO SAMPLES CONTAINING SUBSTANCE QUANTITIES WITHIN THE SPECIFIED INTERVAL. |
| ACCURACY | - DIFFERENCE BETWEEN THE ADDED (THEORETICAL) AND THE RECOVERED (EXPERIMENTAL) AMOUNTS SHOULD BE BETWEEN 80% AND 110% FOR IMPURITY CONCENTRATION <1 µg/mL; BETWEEN 80% AND 110% FOR IMPURITY CONCENTRATION > 1 µg/mL to < 10 µg/mL AND BETWEEN 90% AND 107% FOR IMPURITY CONCENTRATION > 10 µg/mL TO < 100 µg/mL. |
| | - RSD BETWEEN 3 SAMPLES OF EACH CONCENTRATION LEVEL (0.05%, 0.14%, 1.00% AND 2.00% OF THE NOMINAL CONCENTRATION OF CANABIDIOL OF 4000.00 µg/mL) AND IT SHOULD NOT EXCEED 15% FOR IMPURITY CONCENTRATION <1 µg/mL; 11% FOR IMPURITY CONCENTRATION ≥ 1 µg/mL TO <10 µg/mL AND 7.3% FOR IMPURITY CONCENTRATION > 10 µg/mL TO <100 µg/mL. |
| REPEATABILITY | - REPETIBILITY RESULT EVALUATION WILL BE PERFORMED BY ASSESSING THE RELATIVE STANDARD DEVIATION OF EACH LEVEL, THIS SHOULD NOT EXCEED 15% FOR IMPURITY CONCENTRATION <1 µg/mL; 11% FOR IMPURITY CONCENTRATION > 1 µg/mL TO <10 µg/mL AND 7.3% |
| | FOR IMPURITY CONCENTRATION ≥ 10 µg/mL TO <100 µg/mL. |
| INTERMEDIARY ACCURACY | - INTERMEDIATE ACCURACY WILL BE ASSESSED THROUGH THE VARIATION BETWEEN THE MEANS OF THE CONTENTS OBTAINED AT EACH CONCENTRATION LEVEL ON THE FIRST AND ON THE SECOND DAY OF ANALYSIS, WHICH DIFFERENCE SHOULD NOT BE UPPER 15% FOR IMPURITY CONCENTRATION <1 µg/mL; 11% FOR IMPURITY CONCENTRATION ≥ 1 µg/mL TO <10 µg/mL AND 7.3% FOR IMPURITY CONCENTRATION ≥ 10 µg/mL TO <100 µg/mL. |
| ROBUSTNESS | - RESOLUTION BETWEEN THE PEAKS OF DEGRADATION AND THE CANABIDIOL PEAK ON EACH CHROMATOGRAM SHOULD NOT BE LESS THAN 1.5 AND VARIABLES BETWEEN THE CONTENT OF THE ACCURACY SOLUTION AND THE CONTENT OBTAINED WITH THE METHOD WITHOUT CHANGES SHOULD NOT BE UPPER 15.0% WITH RECOVERY OF 80 TO 110% FOR IMPURITY CONCENTRATION <1 µg/mL, SHOULD NOT BE UPPER 11.0% WITH RECOVERY; BETWEEN 80 TO 110% FOR IMPURITY CONCENTRATION ≥ 1 µg/mL TO <10 µg/mL, SHOULD NOT BE UPPER 7.3% WITH RECOVERY BETWEEN 90 TO 107% FOR IMPURITY CONCENTRATION > 10 µg/mL TO <100 µg/mL. |

Validated analytical methodology and acceptable limits to ensure identity, quality and purity of the formulation object of this patent.

| TEST | SPECIFICATIONS | |
|---|---|---|
| DESCRIPTION | OILY LIQUID, BRIGHT AND GOLDEN YELLOW COLOR | |
| IDENTIFICATION | MAIN PEAK RETENTION TIME OBTAINED IN THE TEST SOLUTION CHROMATOGRAM CORRESPONDS TO THE PEAK RETENTION TIME OBTAINED IN THE STANDARD SOLUTION CHROMATOGRAM | |
| DETERMINATION VOLUME | AVERAGE VOLUME | > 30 mL |
| | LOWER LIMIT | > 95.0% |
| RELATED SUBSTANCES | CANNABINOL | 0.20% |
| | DELTA 9 TETRAHYDROCANNABINOL | 0.20% |
| | INESPECIFIC INDIVIDUAL IMPURITIES | 0.20% |
| | TOTAL IMPURITIES | 0.60% |

| DOSING | 180 mg/mL - 220 mg/mL |
|---|---|
| BACTERIA TOTAL COUNT | ≤ 100 CFU/ mL |
| MOULDS AND YEAST TOTAL COUNT | ≤ 10 CFU/ mL |
| *ESCHERICHIA COLI* | ABSENT |

Validation of analytical methodology for formulation content and dosing object of this patent.

| EVALUATED PARAMETER | SPECIFICATIONS |
|---|---|
| SPECIFICITY AND SELECTIVITY | - PROOF THAT THE PEAK QUANTIFIED IN THE CHROMATOGRAM OF THE TEST SOLUTION IS REALLY THE ANALYTE OF INTEREST (CANABIDIOL) BY COMPARING OF THE RETENTION TIME OF THIS PEAK WITH THE RETENTION TIME OF CANABIDIOL IN THE STANDARD SOLUTION. |
| | - PROOF THAT NO SUBSTANCE CO-ELUATES (RESOLUTION EQUAL TO OR MORE THAN 1.5) WITH THE CANABIDIOL PEAK IN THE TEST SOLUTION THROUGH THE RAW MATERIAL SAMPLES ANALYSIS SUBMITTED TO FORCED DEGRADATION AND PLACEBO, AND POSTERIOR VERIFICATION OF CANABIDIOL PEAK PURITY IN THESE SOLUTIONS. THE RESULTS OF THE PEAK PURITY ARE EXPRESSED BY COMPARING THE PURITY ANGLE WITH THE PURITY THRESHOLD WHEN PURITY ANGLE VALUE IS LESS THAN THRESHOLD PURITY VALUE, PEAK IS PURE AND, ON CONTRARY, PEAK IS IMPURE. |
| LINEARITY | - CORRELATION COEFFICIENT (R) MUST BE EQUAL OR GREATER THAN 0.99. |
| | - DO NOT PRESENT TREND IN THE DISTRIBUTION OF RESIDUES. |
| | - RELATIVE STANDARD DEVIATION (RSD) BETWEEN THE 3 SAMPLES OF EACH CONCENTRATION LEVEL, AND IT SHOULD NOT EXCEED 2.0% FOR CONCENTRATION 100 µg/mL TO <1000 µg/mL. |
| INTERVAL | - THE INTERVAL OF THE ANALYTICAL METHODOLOGY WILL BE ESTABLISHED BY THE CONFIRMATION THAT THE METHOD PROVIDES PRECISION, ACCURACY AND LINEARITY SUITABLE WITHIN 80% TO 120% RANGE OF THE NOMINAL CONCENTRATION OF THE SAMPLE IN THE DOSING ANALYTICAL METHODOLOGY (200.0 µg/mL). |
| ACCURACY | - THE RECOVERY AVERAGE IN THREE LEVELS (80%, 100% AND 120% OF THE NOMINAL CONCENTRATION OF CANABIDIOL IN THE SAMPLE) MUST BE BETWEEN 98% AND 102% |
| REPEATABILITY 1^{ST} AND 2^{ND} DAY | RELATIVE STANDARD DEVIATION (RSD) BETWEEN SIX DETERMINATIONS SHOULD BE MINOR OR |
| ACCURACY | EQUAL 2.0%. |
| INTERMEDIARY ACCURACY | - RELATIVE MEAN DIFFERENCE BETWEEN FIRST REPETIBILITY MEANS AND SECOND MUST BE LESS OR EQUAL 2.0% |
| ROBUSTNESS | - METHOD WILL BE CONSIDERED ROBUST FOR SUCH VARIATIONS WHEN THE RESOLUTION BETWEEN THE CANABIDIOL PEAK AND ANY OTHER PEAK IS NOT LESS THAN 1.5 AND THE TEST SOLUTION CONTENT WILL NOT VARY MORE THAN 2.0% OF THE CONTENT OBTAINED WITHOUT CHANGE IN ANALYTICAL METHODOLOGY. |

Validation of analytical methodology for related substances and degradation products of the formulation object of this patent.

| EVALUATED PARAMETER | SPECIFICATIONS |
|---|---|
| SPECIFICITY | - PROOF THAT THE PEAK QUANTIFIED IN THE CHROMATOGRAM OF THE TEST SOLUTION IS REALLY THE ANALYTE OF INTEREST (CANABIDIOL) THROUGH THE COMPARISON OF THE RETENTION TIME OF THIS PEAK WITH THE RETENTION TIME OF CANABIDIOL IN THE STANDARD SOLUTION. |
| | - PROOF THAT ANY DEGRADATION PRODUCT CO-ELUATES (RESOLUTION EQUAL TO OR MORE THAN 1.5) WITH THE CANABIDIOL PEAK, Δ9-THC AND CBN IN THE TEST SOLUTION THROUGH THE SAMPLES ANALYSIS SUBMITTED TO FORCED DEGRADATION IN ACID, BASE, OXIDATIVE, THERMAL, PHOTOLYTIC, HUMIDITY AND METAL ION MEDIUM AND POSTERIOR VERIFICATION OF CANABIDIOL PEAK PURITY IN THESE SOLUTIONS AND POSTERIOR VERIFICATION OF CANABIDIOL PEAK PURITY, Δ9-THC AND CBN (IF FORMED) IN THESE SOLUTIONS. THE RESULTS OF THE PEAK PURITY ARE EXPRESSED BY COMPARING THE PURITY ANGLE WITH THE PURITY THRESHOLD WHEN PURITY ANGLE VALUE IS LESS THAN THRESHOLD PURITY VALUE, PEAK IS PURE AND, ON CONTRARY, PEAK IS IMPURE. |
| LINEARITY | - CORRELATION COEFFICIENT (R) MUST BE EQUAL OR GREATER THAN 0.99. |
| | - DO NOT PRESENT TREND IN THE DISTRIBUTION OF RESIDUES. |
| | - RELATIVE STANDARD DEVIATION (RSD) |
| | BETWEEN THE 3 SAMPLES OF EACH CONCENTRATION LEVEL WILL BE ALSO EVALUATED, AND IT SHOULD NOT EXCEED 15.0% FOR CONCENTRATION < 1 µg/mL, IT SHOULD NOT EXCEED 11.0% FOR CONCENTRATION ≥ 1 µg/mL TO <10 µg/mL AND SHOULD NOT EXCEED 7.3% FOR CONCENTRATION > 10 µg/mL TO <100 µg/mL. |
| RESPONSE FACTOR | - RELATIVE RESPONSE FACTOR WILL BE OBTAINED THROUGH THE RATIO BETWEEN THE ANGULAR COEFFICIENTS FROM IMPURITIES AND ACTIVE, OBTAINED FROM THE LINEARITY PARAMETER. |
| LIMIT OF QUANTIFICATION | - THIS PARAMETER WAS DETERMINED FROM THE RESULTS OF THE LINEARITY PARAMETER. |
| INTERVAL | - THIS PARAMETER IS ESTABLISHED BY THE CONFIRMATION THAT THE METHOD PROVIDES PRECISION, ACCURACY AND LINEARITY SUITABLE WHEN APPLIED TO SAMPLES CONTAINING SUBSTANCE QUANTITIES WITHIN THE SPECIFIED INTERVAL. |
| ACCURACY | - DIFFERENCE BETWEEN THE ADDED (THEORETICAL) AND THE RECOVERED (EXPERIMENTAL) AMOUNTS SHOULD BE BETWEEN 80% AND 110% FOR IMPURITY CONCENTRATION <1 µg/mL; BETWEEN 80% AND 110% FOR IMPURITY CONCENTRATION ≥ 1 µg/mL TO < 10 µg/mL AND BETWEEN 90% AND 107% FOR IMPURITY CONCENTRATION ≥ 10 µg/mL TO < 100 µg/mL. |
| | - RSD BETWEEN 3 SAMPLES OF EACH CONCENTRATION LEVEL (0.05%, 0.2%, 1.00% AND 2.00%) OF THE NOMINAL CONCENTRATION OF CANABIDIOL OF 4000,00 µg/mL) AND IT SHOULD NOT EXCEED 15% FOR IMPURITY CONCENTRATION <1 µg/mL; 11% FOR IMPURITY CONCENTRATION > 1 µg/mL TO <10 µg/mL AND 7.3% FOR IMPURITY CONCENTRATION ≥ 10 µg/mL TO <100 µg/mL. |
| REPEATABILITY | - REPETIBILITY RESULT EVALUATION WILL BE PERFORMED BY ASSESSING THE RELATIVE STANDARD DEVIATION OF EACH LEVEL, THIS SHOULD NOT EXCEED 15% FOR IMPURITY CONCENTRATION <1 µg/mL; 11% FOR IMPURITY CONCENTRATION ≥ 1 µg/mL TO <10 µg/mL AND 7.3% FOR IMPURITY CONCENTRATION > 10 µg/mL |
| | TO <100 µg/mL. |
| INTERMEDIARY ACCURACY | - INTERMEDIATE ACCURACY WILL BE ASSESSED THROUGH THE VARIATION BETWEEN THE MEANS OF THE CONTENTS OBTAINED AT EACH CONCENTRATION LEVEL ON THE FIRST AND ON THE SECOND DAY OF ANALYSIS, WHICH DIFFERENCE SHOULD NOT BE UPPER 15% FOR IMPURITY CONCENTRATION <1 µg/mL; 11% FOR IMPURITY CONCENTRATION ≥ 1 µg/mL TO <10 µg/mL AND 7.3% FOR IMPURITY CONCENTRATION > 10 µg/mL TO <100 µg/mL. |
| ROBUSTNESS | - RESOLUTION BETWEEN THE PEAKS OF DEGRADATION AND THE CANABIDIOL PEAK ON EACH CHROMATOGRAM SHOULD NOT BE LESS THAN 1.5 AND VARIABLES BETWEEN THE CONTENT OF THE ACCURACY SOLUTION AND THE CONTENT OBTAINED WITH THE METHOD WITHOUT CHANGES SHOULD NOT BE UPPER 15.0% WITH RECOVERY OF 80 TO 110% FOR IMPURITY CONCENTRATION <1 µg/mL, SHOULD NOT BE UPPER 11.0% WITH RECOVERY BETWEEN 80 TO 110% FOR IMPURITY CONCENTRATION ≥ 1 µg/mL TO <10 µg/mL, SHOULD NOT BE UPPER 7.3% WITH RECOVERY BETWEEN 90 TO 107% FOR IMPURITY CONCENTRATION ≥ 10 µg/mL TO <100 µg/mL. |

## Claims

1. Process for preparing an oral liquid composition **characterized by** comprising the steps of:
a. Dissolution of cannabidiol in corn oil under stirring and heating at temperatures between 30 °C and 80 °C:
b. Addition of the excipients and homogenization
wherein the excipients are added after cooling of the solution, and kept under stirring until complete solubilization;
wherein the excipients are selected from antioxidant, sweetener, flavouring, preservative or combination thereof; and
wherein the concentration of cannabidiol in the composition ranges from 20 to 250 mg/ml.

## Patentansprüche

1. Verfahren für die Herstellung einer oralen flüssigen Zusammensetzung, die durch Umfassen der Schritte gekennzeichnet ist des:
a. Lösens von Cannabidiol in Maisöl unter Rühren und Erhitzen bei Temperaturen zwischen 30 °C und 80 °C;
b. Zugebens der Trägerstoffe und Homogenisierens,
wobei die Trägerstoffe nach Kühlen der Lösung zugegeben und unter Rühren gehalten werden, bis die Solubilisierung abgeschlossen ist;
wobei die Trägerstoffe unter einem Antioxidationsmittel, Süßungsmittel, Aromastoff, Konservierungsmittel oder einer Kombination davon ausgewählt werden; und
wobei die Konzentration von Cannabidiol in der Zusammensetzung im Bereich von 20 bis 250 mg/ml liegt.

## Revendications

1. Procédé de préparation d'une composition liquide à usage oral, **caractérisé en ce qu'**il comprend les étapes suivantes :
a. Dissolution du cannabidiol dans de l'huile de maïs sous agitation et chauffage à des températures comprises entre 30 °C et 80 °C ;
b. L'ajout des excipients et l'homogénéisation,
dans lequel les excipients sont ajoutés après refroidissement de la solution et maintenus sous agitation jusqu'à solubilisation complète ;
dans lequel les excipients sont choisis parmi un antioxydant, un édulcorant, un arôme, un conservateur ou une combinaison de ceux-ci ; et
dans lequel la concentration en cannabidiol dans la composition est comprise entre 20 et 250 mg/mL.
